# EUROPÄISCHE PATENTSCHRIFT

(11) **EP 0 533 872 B1**
(45) Veröffentlichungstag und Bekanntmachung des Hinweises auf die Patenterteilung: **17.12.1997**
(21) Anmeldenummer: 92906947.4
(22) Anmeldetag: 17.03.1992
(51) Int. Cl.: A61K 38/16

(54) **VERWENDUNG VON APOTRANSFERRIN, WELCHES ZINK- ODER KUPFERIONEN ANGELAGERT HAT, ZUR BEKÄMPFUNG DER TOXISCHEN WIRKUNG DES ENDOTOXINS**
USE OF APOTRANSFERRIN WHICH HAS ACCUMULATED ZINC OR COPPER IONS TO COMBAT THE TOXIC ACTION OF ENDOTOXIN
UTILISATION D'APOTRANSFERRINE AYANT ACCUMULE DES IONS ZINC OU CUIVRE POUR LUTTER CONTRE L'EFFET TOXIQUE DE L'ENDOTOXINE

(30) Priorität: 21.03.1991 DE 4109254
(43) Veröffentlichungstag der Anmeldung: 31.03.1993
(73) Patentinhaber: NITSCHE, Dietrich, D-24105 Kiel (DE)
(72) Erfinder: NITSCHE, Dietrich, D-24105 Kiel (DE)
(74) Vertreter: Tönnies, Jan G., Dipl.-Ing.
(86) Internationale Anmeldenummer: DE9200230
(87) Internationale Veröffentlichungsnummer: WO9216227

(56) Entgegenhaltungen:
- WO-A-91/13629
- DE-A- 3 844 667
- Biological Abstracts, Band 90, Nr. 11, 1990, Philadelphia, PA, US; M.C. McGAHAN: "Copper and aspirin treatment increase the antioxidant activity of plasma", Zusammenfassung Nr. 126999
- Berger et al 1990 Clin.Chim.Acta 189;1-5

## Beschreibung

Die Erfindung betrifft die Verwendung von Apotransferrin, das zweiwertige Zink- oder Kupferionen angelagert hat, zur Herstellung eines Arzneimittels zur Bekämpfung der toxischen Wirkung des Endotoxins.

Apotransferrin ist ein Glykoprotein mit einem durchschnittlichen Molekulargewicht von ca. 8O OOO Dalton.Das Apotransferrin kann vorwiegend dreiwertiges Eisen reversibel binden. Es ist aber auch in der Lage, an Stelle des Eisens andere Metallionen wie z.B. Cu(II), Mn(III), Co(III) oder Zn(II) anzulagern. Im menschlichen und tierischen Organismus ist Apotransferrin vorwiegend als Protein-Metall-Komplex mit dreiwertigem Eisen in Form des Transferrin zu finden.

Als Hauptfunktion des Apotransferrins wird seine Fähigkeit angesehen, spezifisch dreiwertiges Eisen zu binden und zu transportieren. Hierbei kann es ein oder zwei Moleküle Eisen reversibel anlagern. Auf diese Weise wird das Eisen nach seiner Resorption im Dünndarm zu den Eisendepots in der Leber, in der Milz sowie zu den Retikulozyten im blutbildenden Gewebe transportiert. Der Normalbereich für die Konzentration des Apotransferrin-Eisen-Komplex (Transferrin) im Plasma beträgt 2OO mg/dl bis 4OO mg/dl.

Als eine weitere, wichtige Funktion des Transferrins, in seiner eisenfreien Form, wird seine bakteriostatische Eigenschaft angesehen (Martin CM, Jandl JH, Finland M. J Infect Dis 1963; 112: 158 -163; Fletcher J. Immunology 1971; 2O: 493 - 5OO ). Eisen ist ein wesentlicher Wachstumsfaktor für die Bakterien. Durch die Komplexbildung des Eisens mit dem Transferrin wird die Konzentration an freiem Eisen im Plasma unter der Mindestkonzentration gehalten, die für das Wachstum der Bakterien erforderlich ist. Aus dem bakteriostatischen Effekt des Transferrins wurde von Berger und Beger ( Berger D, Beger HG. Clin Chim Acta 1987; 163: 289 - 299; Arzneim-Forsch /Drug Res 1988; 38: 817 - 82O; und Prog Clin Biol Res 1988; 272: 115 - 124 ) geschlossen, daß dem Transferrin in der eisenfreien Form bei ausgedehnten gramnegativen Infektionen sogar eine gewisse Bedeutung als Zusatztherapeutikum für die antimikrobielle Therapie im Sinne einer Ergänzung zu der herkömmlichen Antibiotikatherapie zukommen könnte.

Weiterhin ist Apotransferrin in der Lage, nach Anlagerung von dreiwertigem Eisen, d.h. als Transferrin, die biologische Aktivität der Endotoxine herabzusetzen. Die Fähigkeit des Transferrins zur Endotoxinneutralisation nimmt mit steigendem Eisengehalt zu, worauf in DE 38 42 143 und in DE 38 44 667 ausführlich eingegangen wird. Aus der Tatsache, daß Apotransferrin nach Anlagerung von Eisen-Ionen, je nach Eisengehalt, mehr oder minder in der Lage ist Endotoxin zu neutralisieren, kann nicht zwangsläufig geschlossen werden, daß der gleiche Effekt auch durch Anlagerung von anderen Metall-Ionen erreicht wird.

Im Gegensatz zu der in DE 38 42 143 und in DE 38 44 667 beschriebenen Erkenntnis wird von Berger et al. berichtet, daß nur Apotransferrin in der Lage ist Endotoxin zu neutralisieren ( Berger D, Winter M, Beger HG. Clin Chim Acta 199O; 189: S.1 (Summary):" ....*the endotoxin binding capacity is restricted to apotransferrin*." ) und daß die Fähigkeit von Apotransferrin zur Bindung und Neutralisation von Endotoxin bei Anlagerung von Eisen abnimmt ( Berger D, Beger HG. Clin Chim Acta 1987; 163: 289 - 299; Arzneim-Forsch/Drug Res 1988; 38: 817 - 82O; und Prog Clin Biol Res 1988; 272: 115-124; Langenbecks Archiv für Chirurgie 199O; Suppl. 1-6; Berger D, Winter M, Beger HG. Clin Chim Acta 199O, 189: S.1-6 ). Von den Autoren wird sogar darauf hingewiesen, daß Transferrin die Toxizität von Endotoxin erhöht ( Berger D, Winter M, Beger HG. Clin Chim Acta 199O; 189: S.4: "*transferrin ... exhibits endotoxicity enhancing activity*." ). Von Berger et al. wird weiterhin berichtet, daß neben dem pH auch die Anwesenheit von divalenten Kationen (Ca²⁺, Mg²⁺, Mn²⁺) in dem Reaktionsmedium für eine Bindung des Endotoxins durch Apotransferrin von entscheidender Bedeutung ist( Berger D, Beger HG. Arzneim-Forsch/Drug Res 1988; 38: 817 - 82O; Prog Clin Biol Res 1988; 272: 115-124; Berger D, Winter M, Beger HG. Clin Chim Acta 199O, 189: S.1-6 und Berger D, Kitterer WR, Beger HG. Eur J Clin Invest 1990; 20: 66 - 71). Die Autoren weisen lediglich auf die Notwendigkeit hin, daß divalente Kationen im Reaktionsansatz vorhanden sein müssen, wobei eine Konzentration von 2 mmol/l bzw. 3 mmol/l( Mg²⁺ ) angegeben wird. Ein evtl. Einfluß der Ionenkonzentration auf die Bindung von Endotoxin durch Transferrin wird nicht beschrieben. Die Autoren kommen in den Druckschriften nicht zu dem Schluß, daß eine Steigerung der Ionenkonzentration in der Lösung und eine damit indirekt verbundene Zunahme der Apotransferrin-Kationen-Komplexe zu einer Verbesserung der Bindung des Endotoxins durch Apotransferrin führt. Damit ergeben sich aus diesen Druckschriften keinerlei Hinweise dafür, daß eine primäre Anlagerung der Kationen an das Apotransferrin eine der Voraussetzungen für die Bindung des Endotoxins an das Apotransferrin sein könnte.

Das Endotoxin ist ein Zellwandbestandteil der gramnegativen Bakterien und wird erst durch den Bakterienzerfall freigesetzt. Es ist ein Makromolekül mit einem Molekulargewicht bis maximal 1 x 1O⁶ Dalton. Es setzt sich vorwiegend aus Zuckerverbindungen und Fettsäuren zusammen. Daneben können noch Proteinreste angelagert sein, die von der Bakterienwand stammen. Das Endotoxinmolekül ist aus drei strukturell und immunbiologisch unterschiedlichen Subregionen aufgebaut:
a) Subregion 1, die O-spezifische Kette, besteht aus mehreren, sich wiederholenden Oligosaccharideinheiten, die jeweils von maximal 5 Neutralzuckern gebildet werden. Die Anzahl der Oligosaccharide ist von der Bakterienart abhängig; z.B. hat das Endotoxin von S.abortus equi in dieser Region 8 Oligosaccharid-Einheiten.
b) Subregion 2, das Kernoligosaccharid besteht u.a. aus N-Acetylglucosamin, Glucose, Galactose, Heptose und 2-Keto-3-Desoxyoctonsäure.
c) Subregion 3, das Lipid A ( MG 2OOO Dalton ) besteht aus einem phosphorylierten D-Glukosamin-Disaccharid, an das mehrere - ca. 7 - langkettige Fettsäuren in Amid- und Esterbindung geknüpft sind. Der Träger der toxischen Eigenschaften ist das Lipid A, wobei die toxische Wirkung vorwiegend von Fettsäureresten in dieser Region ausgeht.

Aufgrund der Größe des Endotoxinmoleküls und aufgrund seiner Ladungsverhältnisse können sich verschiedene Verbindungen und Proteine an den vielen Gruppen bzw. Seitenketten der drei verschiedenen Subregionen des Endotoxins anlagern, ohne dadurch irgendeine Beeinflussung der toxischen Eigenschaften des Endotoxins herbeizuführen. Physiologischerweise ist an das Lipid A ein Protein gebunden, das sog. Lipid A-assoziierte Protein. Die Abtrennung dieses Proteinanteils von dem Lipid A führt bei einigen Endotoxinen zu keinerlei Veränderung der toxischen Wirkung. Bei vielen Endotoxinen hat jedoch die Bindung von Protein an das Endotoxin eine wesentliche Steigerung der toxischen Wirkung zur Folge ( Rietschel ET. et al. Bacterial Endotoxins: Chemical Structure, Biological Activity and Role in Septicaemia. Scand J Infect Dis 1982; Suppl. 31: 8 - 21). Beispielsweise wurde beobachtet, daß bestimmte Endotoxine, die einen Proteinanteil angelagert haben, ca.1OO-fach toxischer sein können als Endotoxine, von denen der Proteinanteil abgetrennt wurde ( Morrison DC, Oudes ZG, Betz J. (198O): The role of lipid A and lipid A-associated protein in cell degranulation mechanisms. In: Eaker D. Wadstrom T (Eds). NATURAL TOXINS, pp 287 -294, Pergamon Press. New York ). Auch bei tierexperimentellen Untersuchungen wurde bei verschiedenen Endotoxinen dann eine Abnahme der toxischen Wirkung beobachtet, wenn der Proteinanteil abgespalten war ( Hitchcock PJ, Morrison DC. (1984) "The protein component of bacterial endotoxins". In: E.T. Rietschel (editor) HANDBOOK of ENDOTOXINS, Vol 1: Chemistry of Endotoxin, pp 339-375, Elsevier Science Publishers B.V. ).

Beim Gesunden kann normalerweise kein freies, d.h. biologisch aktives Endotoxin im Blut nachgewiesen werden. Bei folgenden krankhaften Zuständen kann jedoch vermehrt biologisch aktives Endotoxin im Blut auftreten:
a) bei einem gesteigerten Übertritt von Endotoxin aus dem Intestinaltrakt, aufgrund von Permeabilitätsstörungen der Darmwand wie z.B. bei einer schweren Enterocolitis, im Schock, nach Traumen oder aufgrund einer vermehrten Freisetzung im Verlaufe einer enteralen Antibiotikatherapie;
b) bei einer Störung der Endotoxinelimination über die Leber, z.B. bei Leberparenchymschäden oder nach Leberteilresektion.
c) bei einer gesteigerten Endotoxin-Freisetzung von einem größeren gramnegativen Infektionsherd, wie es im Rahmen einer Therapie mit einem Antibiotikum zu beobachte ist.

Zum Schutz vor einer toxischen Organschädigung hat das Plasma des Gesunden die Fähigkeit, das ständig aus dem Intestinaltrakt übertretende Endotoxin zu inaktivieren. Bei einem starken Endotoxinübertritt in das Blut erschöpft sich die Fähigkeit des Plasmas zur Inaktivierung des Endotoxins rasch, sodaß vermehrt biologisch aktives Endotoxin im Plasma auftritt, welches dann zu dem klinischen Bild der Endotoxinämie führt. Bei längerer Dauer kann die Endotoxinämie zum Zelluntergang und damit schließlich zum Organversagen führen. Aus diesen Gründen sind bei allen Erkrankungen, bei denen ein vermehrter Endotoxinübertritt in das Blut zu erwarten ist, sowie bei Erkrankungen, bei denen die physiologische Endotoxinelimination über die Leber gestört ist, zusätzliche therapeutische Maßnahmen erforderlich, um die Endotoxinaktivität im Blut herabzusetzen.

Bis zu einem gewissen Grad kann die Endotoxinaktivität im Plasma durch die Gabe polyvalenter 7S-IgG-Präparationen herabgesetzt werden, was vermutlich auf deren Gehalt an Lipid A-Antikörper zurückzuführen ist. Eine weitere Verbesserung der Endotoxinneutralisation wird durch die Anreicherung der IgG-Präparation mit Immunglobulinen der IgM-Fraktion erreicht, in der ein höherer Lipid A-Antikörper Titer vorhanden ist ( Appelmelk BJ et al., Microbiol Pathogenesis 1987; 2: 391 - 393 )

Wie experimentelle und klinische Untersuchungen ( Baumgartner JD, et al. *Prevention of gram-negative shock and death in surgical patients by antibody to endotoxin core glycolipid.* Lancet 1985;ii: 59-63; Dunn DL, Priest BP, Condie RM. *Protective capacity of polyclonal and monoclonal antibodies directed against endotoxin during experimental sepsis.* Arch Surg 1988; 123: 1389 - 1393; Ziegler EJ. et al. *Treatment of gram-negative bacteremia and septic shock with HA-1A human monoclonal antibody against endotoxin.* N Engl J Med 1991, 324: 429-436) gezeigt haben, ist eine Verbesserung der Letalität bei der Sepsis durch die Herabsetzung der Endotoxinaktivität im Plasma möglich, wenn monoklonale Antikörper appliziert werden, die gegen das **"** ***core*****"** oder gegen das Lipid A des Endotoxins gerichtet sind.

Eine Alternative zur Neutralisation von Endotoxin durch monoklonale Antikörper stellt die Neutralisation der toxischen Wirkung des Endotoxins durch Transferrin dar (DE 38 44 667). Durch die Kombination von Transferrin mit polyvalenten Immunglobulin-Präparationen kann hierbei ein synergistischer Effekt erreicht werden, wie in DE 38 42 143 beschrieben.

Wie in DE 38 44 667 ausführlich dargelegt, nimmt die Fähigkeit von Apotransferrin zur Neutralisation von Endotoxin mit steigendem Gehalt an dreiwertigem Eisen zu. Im Organismus kann jedoch das Eisen durch Reduktion zu Fe²⁺ wieder vom Transferrin abgespalten werden. Da die Sauerstoffaktivierung enzymgekoppelter Reaktionen, d.h. die Bildung von Sauerstoffradikalen durch die neutrophilen Granulocyten durch ein erhöhtes Angebot von Fe²⁺-Ionen stimuliert werden kann ist nicht auszuschließen, daß die unerwünschte Bildung von schädigenden Sauerstoffradikalen im Organismus durch die Therapie mit Transferrin möglicherweise begünstigt wird. Aber auch nichtenzymatische Prozesse, die zur Sauerstoffaktivierung und der damit verbundenen Bildung von schädigenden Sauerstoffradikalen führen, wie die sog Haber-Weiss-Reaktion ( Haber F., Weiss J. Proc Royal Soc [A] 1934; 147: 332-351) können durch das vom Transferrin abgespaltene und reduzierte zweiwertige Eisen katalysiert werden (Carlin G., Djursäter R. FEBS Lett 1984; 177: 27-3O). Außerdem kann es bei der Sepsis durch die erhöhte Proteasen-Aktivität im Plasma auch zu einer Spaltung des Transferrin-Moleküls kommen ( Esparza I., Brock JH. Biochem Biophys Acta 198O; 622:297-3O4; Doring GM. et al. Infect Immun 1988; 56: 291-293 ). Die hierbei frei werdenden Fragmente des Transferrin, die Eisen angelagert haben, können dann ebenfalls die Sauerstoff-Radikalbildung durch die neutrophilen Granulocyten katalysieren ( Bradley EB., Edeker BL. J Clin Invest 1991; 88: 1O92-11O2 ). Die Sauerstoffradikale können einerseits die Zellmembran direkt schädigen und andererseits durch Steigerung der Prostaglandinsynthese auch indirekt zu einer zusätzlichen Schädigung des Organismus führen.

Der Erfindung liegt die Aufgabe zugrunde ein Mittel zu finden, welches in der Lage ist, einerseits die biologische Wirkung des Endotoxins in ausreichendem Maße herabzusetzen und sich auf diese Weise als effizientes Therapeutikum für die Bekämpfung der toxischen Wirkung der Endotoxine eignet. Gleichzeitig soll es jedoch auch in der Lage sein, die unerwünschte Bildung schädigender Sauerstoffradikale bei der Endotoxinämie gering zu halten.

Erfindungsgemäß wird diese Aufgabe durch die Verwendung von Apotransferrin gelöst, welches zweiwertige Zink- oder Kupfer-Ionen angelagert hat. Diese Apotransferrin-Metall-Komplexe eignen sich auch für die Kombination mit Immunglobulin-Präparationen sowie für den Zusatz zu einer Plasma-Protein-Lösung bzw. zu einer Serumkonserve.

Zur Prüfung in welchem Ausmaß Endotoxin durch die Komplexe von Apotransferrin mit zweiwertigen Zink- oder Kupfer-Ionen, sowie durch die Kombination dieser Komplexe mit Immunglobulinen inaktiviert werden kann, wurden Untersuchungen in vitro und in vivo durchgeführt.

### Untersuchungen in vitro:

### A) Quantitative Untersuchungen zum Einfluß auf die biologische Aktivität:

Apotransferrin-Zink und Apotransferrin-Kupfer sowie Apotransferrin-Zink oder -Kupfer in Kombination mit Immunglobulinen, wurden mit steigenden Endotoxinmengen jeweils 6O Minuten bei 37°C inkubiert. Nach Abschluß der Inkubation erfolgte die quantitative Bestimmung der biologischen Aktivität des Endotoxins im Überstand mit einer Modifikation des Limulus-Test, unter Verwendung eines chromogenen Substrates ( Nitsche et al. In: Watson SW., Levin J., Novitsky TJ.(eds) DETECTION OF BACTERIAL ENDOTOXINS WITH THE LIMULUS AMEBOCYTE LYSATE TEST; 1987, pp 417 - 429 ). Die untere Nachweisgrenze liegt bei dieser Modifikation des Limulus-Tests bei 1 EU/dl. Die mit dem Limulus-Test erfaßte Endotoxinaktivität korreliert mit der biologischen Aktivität des Endotoxins (Nitsche D., Ulmer A., Flad HD.: Endotoxin determination by the Limulus-Test and the biological activity of endotoxin. Does a correlation exist? - In Vorbereitung - ). Eine Beeinflussung der Limulus-Reaktion durch Zn²⁺- oder Cu²⁺-Ionen konnte im Rahmen von Voruntersuchungen nicht beobachtet werden.

Das Apotransferrin, welches für die Untersuchungen verwendet wurde, war weitgehend frei von Metallionen ( Atf.-0 ). Hier konnte bei der Zinkbestimmung mit Hilfe der Atomabsorptionsspektroskopie kein Zink gefunden werden. Kupfer konnte ebenfalls nicht in den Proben nachgewiesen werden. Auch bei der Eisenbestimmung mit der von Megraw und Bouda ( Megraw RE., Hritz AM., Babson AL., Carroll JJ. Clin Biochem 1973; 6: 266; Bouda J. Clin Chem Acta 1968; 21:159 ) beschriebenen Methode konnte in der 5% Apotransferrin-Lösung kein Eisen nachgewiesen werden. Bei dieser Methode liegt die untere Nachweisgrenze für Eisen bei O,3 µg/dl.

Das Apotransferrin ( Atf.-0 ) wurde mit unterschiedlichen Mengen Zinkchlorid ( ZnCl₂ ) versetzt, wodurch Apotransferrin-Zink-Lösungen mit einem Zinkgehalt von 4,8 µg Zink pro Gramm Apotransferrin (= Atf.-Zn(A) ) sowie Apotransferrin mit einem Zinkgehalt von 598 µg Zink pro Gramm Apotransferrin (= Atf.-Zn(B)) erhalten wurden.

Weiterhin wurde Apotransferrin( Atf.-0 ) mit unterschiedlichen Mengen Kupfer(II)-Chlorid ( CuCl₂ ) versetzt, wodurch Apotransferrin-Kupfer-Lösungen mit einem Kupfergehalt von 57 µg Kupfer pro Gramm Apotransferrin (= Atf.-Cu(A) ) sowie Apotransferrin-Kupfer (= Atf.-Cu(B)) mit einem Kupfergehalt von 74O µg pro Gramm Apotransferrin erhalten wurden.

Zum Vergleich wurde Apotransferrin-Eisen verwendet. Hierzu wurde das Apotransferrin (Atf-0) mit Eisen(III)-Chlorid ( FeCl₃ ) versetzt, derart, daß eine Apotransferrin-Eisen-Lösung (= Trf.-Fe ) mit einem Eisengehalt von 598 µg Eisen pro Gramm Transferrin erhalten wurde.

Folgende Immunglobuline wurden verwendet: 7S-IgG (Sandoglobin^{R}) sowie 7S-IgG, angereichert mit 12% IgM (Pentaglobin^{R}).

Von folgenden Bakterienstämmen wurden die Endotoxine verwendet:
Salmonella abortus equi ( NOVOPYREXAL^{R} ), Pseudomonas aeruginosa Fisher Typ 7, sowie der FDA Endotoxinstandard EC-5 von E.coli O113:H1O:KO.

### BESTIMMUNG DER " MITTLEREN PROZENTUALEN INAKTIVIERUNG".

Die prozentuale Inaktivierung der jeweils untersuchten Endotoxinmenge wurde aus der Differenz zwischen der Endotoxinkonzentration, die zugesetzt worden war und der Endotoxinaktivität bestimmt, die nach der Inkubation mit dem Protein gemessen wurde. Aus den Werten, die auf diese Weise für die verschiedenen Endotoxinkonzentrationen gefunden wurden, wurde für die jeweilige Proteinkonzentration die **" *mittlere******prozentuale In*****aktivierung****"** für den gesamten Endotoxinkonzentrationsbereich (1O EU/dl - 1OOO EU/dl ) berechnet, der untersucht worden war.

### BESTIMMUNG DER INAKTIVIERUNGSKAPAZITÄT FÜR ENDOTOXIN :

Um die Fähigkeit von Apotransferrin-Kupfer, Apotransferrin-Zink bzw. Apotransferrin-Eisen sowie von der Kombination von Apotransferrin-Zink oder Apotransferrin-Kupfer mit Immunglobulinen zur Inaktivierung von Endotoxin zu quantifizieren, wurde die " *INAKTIVIERUNGSKAPAZITÄT* " bestimmt. Aus Gründen der Meßgenauigkeit wurde die Bestimmung dieser Größe nicht im Äquivalenzbereich, sondern bei einem Überschuß von 1O EU/dl und 1OO EU/dl Endotoxin durchgeführt. Für die Berechnung dieser Parameter wurde diejenige Endotoxinmenge [EU/dl] ermittelt, die der jeweiligen Proteinpräparation zugesetzt werden muß, damit die Konzentration der freien Endotoxine im Überstand nach Inkubation ( 6O Minuten, 37°C ) mit der Proteinpräparation noch 1O EU/dl bzw. 1OO EU/dl beträgt. Diese Endotoxinkonzentration wurde aus der Beziehung berechnet, die zwischen der zugesetzten Endotoxinmenge und der nach der Inkubation im Überstand gemessenen Endotoxinkonzentration für die jeweilige Präparation ( Apotransferrin-Zink, Apotransferrin-Kupfer, Immunglobulin und Kombination von Apotransferrin-Zink oder - Kupfer und Immunglobulin ) gefunden wurde. Zur Bestimmung der Inaktivierungskapazität wurde eine Meßserie durchgeführt, bei der Endotoxin in steigender Konzentration [1O EU/dl, 25 EU/dl, 5O EU/dl, 1OO EU/dl, 2OO EU/dl,3OO EU/dl, 5OO EU/dl sowie 75O EU/dl und 1OOO U/dl] mit den verschiedenen Protein-Präparationen 60 Minuten inkubiert wurde. Die Konzentration von Apotransferrin-Kupfer oder Apotransferrin-Zink sowie die Immunglobulinkonzentration im Reaktionsansatz betrug jeweils 312,5 mg/dl. Bei Untersuchung der Kombination von Apotransferrin-Zink oder Apotransferrin-Kupfer und Immunglobulin betrug die Konzentration von jedem Proteinanteil im Reaktionsansatz jeweils 312 mg/dl.

Die auf diese Weise ermittelte Endotoxinmenge stellt nach Abzug des vorgegebenen Endotoxinüberschuß von 1O EU/dl bzw. 1OO EU/dl die mittlere Endotoxinaktivität[EU/dl] dar, die von der entsprechenden Proteinlösung[mg/dl] unter den jeweiligen Bedingungen inaktiviert werden kann. Bezogen auf eine Gesamtproteinkonzentration von 1OO mg/dl stellt diese Endotoxinaktivität ein Maß für die **"** ***mittlere Inaktivierungskapazität*** **[ EU/1OO mg ] "** des jeweiligen Apotransferrin-Metall-Komplex bzw. der Kombination des Apotransferrin-Metall-Komplex mit Immunglobulin dar.

### ERGEBNISSE:

1. Apotransferrin-Metall-Komplex:
   Bei Inkubation von Endotoxin mit Apotransferrin (Atf.-0), welches frei von Metallionen ist, findet sich je nach Art des Endotoxins und der Endotoxinkonzentration eine mittlere Abnahme der Endotoxinaktivität von 32,2% bis 43,6%.
   Bei Inkubation von Endotoxin mit Apotransferrin-Zink ( Atf.-Zn(A) ), welches einen Zinkgehalt von 4,8 µg pro Gramm Apotransferrin hat, kommt es in Abhängigkeit von der Endotoxinspezies und der zugegebenen Endotoxinmenge zu einer mittleren Abnahme der Endotoxinaktivität von 72,5% bis 82,4%.
   Bei einem Zinkgehalt des Apotransferrin-Zink (Atf.-Zn(B)) von 598 µg pro Gramm Apotransferrin beträgt die Reduzierung der Endotoxinaktivität nach der Inkubation je nach Endotoxinspezies und zugegebener Endotoxinmenge: 91,4% bis 97,8%.
   Bei Inkubation von Endotoxin mit Apotransferrin-Kupfer ( Atf.-Cu(A) ), welches einen Kupfergehalt von 57 µg pro Gramm Apotransferrin hat, kommt es in Abhängigkeit von der Endotoxinspezies und der zugegebenen Endotoxinmenge zu einer mittleren Abnahme der Endotoxinaktivität von 84% bis 89,3%.
   Bei einem Kupfergehalt des Apotransferrin-Kupfer (Atf.-Cu(B)) von 74O µg pro Gramm Apotransferrin beträgt die mittlere Abnahme der Endotoxinaktivität je nach Endotoxinspezies und zugegebener Endotoxinmenge 9O,8% bis 96,7%.
   Bei Inkubation von Endotoxin mit Apotransferrin-Eisen ( Trf.-Fe ), welches einen Eisengehalt von 598 µg pro Gramm Apotransferrin hat, kommt es je nach Endotoxinspezies und zugegebener Endotoxinmenge zu einer mittleren Abnahme der Endotoxinaktivität von 92,2% bis 97,6%.
   a) Bezogen auf einen Endotoxinüberschuß von 1O EU/dl beträgt die Inaktivierungskapazität:
      I.) bei Atf.-0 für S.abortus equi: 1,8 EU/1OO mg; für E.coli: 2,3 EU/1OO mg und für Pseudomonas aeruginosa: 2,7 EU/1OO mg.
      II.) bei Atf.-Zn(A) für S.abortus equi: 11,1 EU/1OO mg; für E.coli: 16,3 EU/1OO mg und für Pseudomonas aeruginosa: 14,9 EU/1OO mg.
      III.) bei Atf.-Zn(B) für S.abortus equi: 66,8 EU/1OO mg; für E.coli: 87,02 EU/1OO mg und für Pseudomonas aeruginosa: 72,49 EU/1OO mg.
      IV.) bei Atf.-Cu(A) für S.abortus equi: 34,6 EU/1OO mg; für E.coli: 43,8 EU/1OO mg und für Pseudomonas aeruginosa: 39,7 EU/1OO mg.
      V.) bei Atf.-Cu(B) für S.abortus equi: 7O,1 EU/1OO mg; für E.coli: 79,4 EU/1OO mg und für Pseudomonas aeruginosa: 64,6 EU/1OO mg.
      VI.) bei Trf.-Fe für S.abortus equi: 76,7 EU/1OO mg; für E.coli: 78,2 EU/1OO mg und für Pseudomonas aeruginosa: 73,8 EU/1OO mg.
   b) Bezogen auf einen Endotoxinüberschuß von 1OO EU/dl beträgt die Inaktivierungskapazität:
      I.) bei Aft.-0 für S. abortus equi: 17,8 EU/1OO mg; für E.coli: 19,4 EU/1OO mg und für Pseudomonas aeruginosa: 2O,6 EU/1OO mg.
      II.) bei Atf.-Zn(A) für S. abortus equi: 109.7 EU/1OO mg, für E.coli: 137,4 EU/1OO mg und für Pseudomonas aeruginosa: 145,5 EU/1OO mg.
      III.) bei Atf.-Zn(B) für S. abortus equi: 507,6 EU/1OO mg, für E. coli: 578,5 EU/1OO mg und für Pseudomonas aeruginosa: 647,3 EU/1OO mg.
      IV.) bei Atf.-Cu(A) für S. abortus equi: 362 EU/1OO mg, für E. coli: 387,4 EU/1OO mg und für Pseudomonas aeruginosa: 4O9 EU/1OO mg.
      V.) bei Atf.-Cu(B) für S. abortus equi: 522,4 EU/1OO mg, für E. coli: 527 EU/1OO mg und für Pseudomonas aeruginosa: 585,1 EU/1OO mg.
      VI.) bei Trf.-Fe für S. abortus equi: 418,7 EU/1OO mg, für E.coli: 571,1 EU/1OO mg und für Pseudomonas aeruginosa: 643,9 EU/1OO mg.
   2.) Apotransferrin-Metall-Komplex und 7S-IgG
      Die Kombination von **Apotransferrin-Zink** bzw. von **Apotransferrin-Kupfer** mit einer **7S-IgG** Präparation führt zu einer Steigerung der Inaktivierungskapazität für Endotoxin.
      Bei Inkubation von Endotoxin mit Apotransferrin (312 mg/dl) und 7S-IgG ( 312 mg/dl) wird die Endotoxinaktivität bei Verwendung von Apotransferrin( Atf.-0 ) welches frei von Zink, Kupfer und Eisen ist, bei Endotoxinkonzentrationen unter 1OO EU/dl um maximal 61 % bis 68 % reduziert. Bei höheren Endotoxinkonzentrationen wird die Endotoxinaktivität durch diese Kombination nur noch um ca. 47,1% bis 54,9% herabgesetzt.
      Wird stattdessen Apotransferrin-Zink ( Atf.-Zn(A) ), welches einen Zinkgehalt von 4,8 µg pro Gramm Apotransferrin hat, mit der 7S-IgG-Präparation kombiniert, dann beträgt die Reduzierung der Endotoxinaktivität je nach Endotoxinspezies: 83,1% bis 89,1%.
      Durch die Kombination der 7S-IgG-Präparation mit Apotransferrin-Zink ( Atf.-Zn(B) ), das einen Zinkgehalt von 598 µg pro Gramm Apotransferrin hat, wird erreicht, daß die Endotoxinaktivität je nach Endotoxinspezies um 94,4% bis 97,8% reduziert wird.
      Wenn 7S-IgG in Kombination mit Apotransferrin-Kupfer(Atf.-Cu(A)) verwendet wird, welches einen Kupfergehalt von 57 µg pro Gramm Apotransferrin hat, dann beträgt die Herabsetzung der Endotoxinaktivität je nach Endotoxinspezies: 87,2 % bis 93,1 %.
      a) Wird ein Endotoxinüberschuß von 1O EU/dl zugrunde gelegt, dann beträgt die Inaktivierungskapazität:
         I.) bei Atf.-0 und 7S-IgG für S.abortus equi: 6,1 EU/1OO mg, für E.coli: 1O,6 EU/1OO mg und für Pseudomonas aeruginosa: 11,4 EU/1OO mg.
         II.) bei Atf.-Zn(A) und 7S-IgG für S.abortus equi: 24,2 EU/1OO mg, für E.coli: 3O,1 EU/1OO mg und für Pseudomonas aeruginosa: 27,5 EU/1OO mg.
         III.) bei Atf.-Zn(B) und 7S-IgG für S.abortus equi: 74,1 EU/1OO mg, für E.coli: 98,9 EU/1OO mg und für Pseudomonas aeruginosa: 86,8 EU/1OO mg.
         IV.) bei Atf.-Cu(A) und 7S-IgG für S.abortus equi: 40,1 EU/1OO mg, für E.coli: 50,6 EU/1OO mg und für Pseudomonas aeruginosa: 46,3 EU/1OO mg.
      b) Bezogen auf einen Endotoxinüberschuß von 1OO EU/dl beträgt die Inaktivierungskapazität:
         I.) bei Atf.-0 und 7S-IgG für S.abortus equi: 28,6 EU/ 1OO mg, für E.coli: 3O,7 EU/1OO mg und für Pseudomonas aeruginosa: 39,1 EU/1OO mg.
         II.) bei Atf.-Zn(A) und 7S-IgG für S.abortus equi: 151,5 EU/1OO mg, für E. coli: 177,9 EU/1OO mg und für Pseudomonas aeruginosa: 195,5 EU/1OO mg.
         III.) bei Atf.-Zn(B) und 7S-IgG für S.abortus equi: 538,4 EU/1OO mg, für E.coli: 602,7 EU/1OO mg und für Pseudomonas aeruginosa: 675,1 EU/1OO mg.
         IV.) bei Atf.-Cu(A) und 7S-IgG für S.abortus equi: 554,7 EU/1OO mg, für E.coli: 547,3 EU/1OO mg und für Pseudomonas aeruginosa: 608,1 EU/1OO mg.
   3.) Apotransferrin-Zink und IgG/A/M (12% IgM)
      Bei Zusatz von **Apotransferrin-Zink** zu einer IgG-Präparation, die mit IgA sowie mit 12 % IgM angereichert ist **(IgG/A/M)**, wird eine weitere Steigerung der Inaktivierungskapazität erreicht. Die Abnahme der Endotoxinaktivität beträgt bei Inkubation von Endotoxin mit der Kombination aus IgG/A/M (12% IgM ) ( 312 mg/dl ) und Apotransferrin-Zink ( 312 mg/dl ) bei Verwendung von Apotransferrin (Atf.-Zn(A)) mit einem Zinkgehalt von 4,8 µg pro Gramm Apotransferrin, je nach Art des Endotoxins 84,7% bis 92,6%.
      Bei Kombination von IgG/A/M mit Apotransferrin (Atf.-Zn(B)), welches einen Zinkgehalt von 598 µg Zink pro Gramm Apotransferrin hat, wird die Endotoxinaktivität, je nach Endotoxinspezies um 94,8% bis 98,1% herabgesetzt.
      a) Bezogen auf einen Endotoxinüberschuß von 1O EU/dl beträgt die Inaktivierungskapazität:
         I.) bei Atf.-Zn(A) und IgG/A/M für S.abortus equi: 26,9 EU/1OOmg, für E.coli: 31,5 EU/1OO mg und für Pseudomonas aeruginosa: 24,9 EU/1OO mg.
         II.) bei Atf.-Zn(B) und IgG/A/M für S. abortus equi: 93,8 EU/1OO mg, für E.coli: 108,5 EU/1OO mg und für Pseudomonas aeruginosa: 97,8 EU/1OO mg.
      b) Für einen Endotoxinüberschuß von 1OO EU/dl beträgt die Inaktivierungskapazität:
         I.) bei Atf.-Zn(A) und IgG/A/M für S.abortus equi: 141,1 EU/1OO mg, für E.coli: 173,2 EU/1OO mg und für Pseudomonas aeruginosa: 192,5 EU/1OO mg.
         II.) bei Atf.-Zn(B) und IgG/A/M für S.abortus equi: 688 EU/1OO mg, für E.coli: 828 EU/1OO mg und für Pseudomonas aeruginosa: 796,9 EU/1OO mg.

### B) EINFLUSS AUF DIE FREISETZUNG DER MEDIATOREN IL-1 UND IL-6

Als weiterer Parameter für den Einfluß der Komplexe von zweiwertigen Zink-Ionen mit Apotransferrin auf die biologische Aktivität des Endotoxins wurde die Freisetzung von IL-1 und IL-6 aus Monocyten herangezogen. Hierzu wurde Endotoxin zunächst mit Apotransferrin-Zink oder mit der Kombination von Apotransferrin-Zink und Immunglobulin inkubiert (6O Min, 37°C). Im Anschluß daran wurde der Überstand mit Monocyten inkubiert und die Konzentration der aus den Monocyten freigesetzten Mediatoren **Interleukin-1** und **Interleukin-6** im Überstand bestimmt. Es wurde Apotransferrin-Zink (Atf.-Zn(A)) verwendet, welches einen Zinkgehalt von 4,8 µg pro Gramm Apotransferrin hat, sowie Apotransferrin-Zink( Atf.-Zn(B) ) mit einem Zinkgehalt von 598 µg pro Gramm Apotransferrin.

Folgende Immunglobulin-Präparation wurde verwendet: 7S-IgG(IgG/A/M), angereichert mit 12% IgM (Pentaglobin^{R})

Von folgenden Bakterienstämmen wurden Endotoxine verwendet: Salmonella abortus equi ( NOVOPYREXAL^{R} ), sowie der FDA Endotoxinstandard EC-5 von E.coli O113:H1O:KO.

Apotransferrin-Zink sowie Apotransferrin-Zink in Kombination mit Immunglobulin wurden mit Endotoxin 6O Minuten bei 37°C inkubiert. Als Kontrollprotein wurde hitzedenaturiertes ( 80°C, 10 Min. ) 7S-IgG mit jeweils identischem Zinkgehalt (4,6 µg bzw. 598 µg Zink pro Gramm 7S-IgG) verwendet. Die Konzentration des zu untersuchenden Proteins im Ansatz betrug 312 mg/dl. Die Inkubation wurde mit Endotoxinkonzentrationen von 5O EU/dl bis 5OO EU/dl durchgeführt.

Nach der Inkubation wurde die jeweilige Endotoxinlösung mit mononukleären Zellen von gesunden Spendern inkubiert ( 16 Std. bei 37°C ). Anschließend wurde die Konzentration der freigesetzten Mediatoren IL-1 und IL-6 bestimmt. Die Bestimmung von IL-1 erfolgte mit Hilfe des Fibroblasten-Proliferationstest ( Loppnow H; Flad HD., Ulmer AJ. et al. "*Detection of* *Interleukin 1 with Human Dermal Fibroblasts*." Immunbiol 1989; 179: 283 - 291 ), unter Verwendung einer EL4-6.1 Thymomzell-Linie. Die Konzentration von **IL-6** wurde ebenfalls über einen Proliferationstest unter Verwendung eines IL-6 abhängigen murinen Hybridoms (7TD1) bestimmt ( Van Damme J., Cayphas S. et al. Eur J Biochem 1987; 168: 543 - 55O; sowie Van Oers MHJ., Van der Heyden A., Aarden LA. Clin exp Immunol 1988; 71: 314 - 419 ).

### ERGEBNISSE:

Durch die Inkubation von Endotoxin mit Apotransferrin-Zink wird die durch das Endotoxin induzierte Freisetzung von Mediatoren aus den mononukleären Zellen reduziert. Dieser Effekt ist vom Zinkgehalt des Apotransferrins abhängig und zwar derart, daß er durch Erhöhung des Zinkgehaltes gesteigert werden kann. Durch die Kombination von Apotransferrin-Zink mit Immunglobulinen kann der hemmende Effekt des Apotransferrin-Zink-Komplex auf die Freisetzung von Mediatoren noch verstärkt werden.
I) KONTROLLPROTEIN ( hitzeinaktiviertes 7S-IgG )
   Nach Inkubation von Endotoxin( 5OO EU/dl ) mit dem hitzeinaktiviertem und mit Zink versetztem 7S-IgG, welches als Kontrollprotein herangezogen wurde, fand sich kein Unterschied in der Höhe der freigesetzten Mediatoren zwischen dem Kontrollprotein mit einem Zinkgehalt von 4,6 µg pro Gramm (Zn(A)) und dem Kontrollprotein mit einem Zinkgehalt von 598 µg pro Gramm (Zn(B)).
   Nach Inkubation des Kontrollprotein mit Endotoxin von S.abortus equi betrug die mittlere Freisetzung von **IL-1** : 2685 U/ml und nach Inkubation mit Endotoxin von E.coli: 2968 U/ml.
   Die Freisetzung von **IL-6** betrug nach Inkubation mit dem Endotoxin von S.abortus equi: 3621 U/ml und nach Inkubation mit Endotoxin von E.coli: 5974 U/ml.
II.) Apotransferrin-Zink
   a) Nach Inkubation von 5OO EU/dl Endotoxin mit Atf.-Zn(A) betrug die mittlere Freisetzung von IL-1 durch das Endotoxin von S.abortus equi: 321 U/ml und durch das Endotoxin von E.coli: 412 U/ml.
      Die Freisetzung von IL-6 betrug nach Inkubation des Kontrollprotein mit Endotoxin von S.abortus equi: 1132 U/ml und mit dem Endotoxin von E.coli: 1825 U/ml.
   b) Nach Inkubation von 5OO EU/dl Endotoxin mit Atf.-Zn(B) betrug die mittlere Freisetzung von IL-1 bei dem Endotoxin von S.abortus equi: 102 U/ml und bei dem Endotoxin von E.coli: 47 U/ml.
      Nach Inkubation mit dem Endotoxin von S.abortus equi betrug die mittlere Freisetzung von IL-6: 526 U/ml und nach Inkubation mit E.coli: 316 U/ml.
III.) Apotransferrin-Zink und IgG/A/M (12% IgM)
   Der Zusatz von Apotransferrin-Zink zu einer 7S-IgG Präparation, die mit IgA sowie mit 12% IgM angereichert ist (IgG/A/M), führte bei einer Immunglobulin-Gesamtkonzentration von 312 mg/dl und einer Apotransferrin-Zink-Konzentration, die ebenfalls 312 mg/dl betrug, nach Inkubation mit 5OO EU/dl Endotoxin:
   a) bei Kombination von IgG/A/M mit Atf.-Zn(A) zu einer Freisetzung von IL-1 von 227 U/ml bei dem Endotoxin von S.abortus equi und mit dem Endotoxin von E.coli zu einer Freisetzung von 267 U/ml IL-1.
      Die IL-6 Menge, die nach der Inkubation freigesetzt wurde, betrug bei dem Endotoxin von S.abortus equi: 872 U/ml und bei E.coli: 631 U/ml.
   b) Bei Kombination von Atf.-Zn(B) mit IgG/A/M (12%) betrug die mittlere Freisetzung von IL-1 nach Inkubation mit S.abortus equi: 31 U/ml und nach Inkubation mit E.coli: 17 U/ml.
      Die mittlere Freisetzung von IL-6 betrug unter diesen Bedingungen bei Inkubation mit S.abortus equi: 186 U/ml und bei Inkubation mit E.coli: 137 U/ml.

### C) Einfluß der Apotransferrin-Komplexe mit Zink- oder Kupfer-Ionen auf die Endotoxinaktivität im Plasma.

Humanplasma von gesunden Spendern wurde 1:1O verdünnt und durch Erhitzen [ 8O°C, 1O Min ] inaktiviert. Anschließend wurde es mit Apotransferrin-Zink oder Apotransferrin-Kupfer versetzt. Die Konzentration von Apotransferrin-Zink betrug im Plasma 28O mg/dl und die Konzentration von Apotransferrin-Kupfer 284 mg/dl. Den Kontrollproben wurde die gleiche Menge Albumin-Zink bzw. Albumin-Kupfer mit jeweils identischem Zink- oder Kupfergehalt zugesetzt. Zu dem mit Apotransferrin-Zink oder -Kupfer bzw. Albumin-Zink oder -Kupfer versetztem Plasma wurde dann Endotoxin ( Pseudomonas-Endotoxin, E.coli-Endotoxin ) zugegeben, sodaß im Ansatz eine Endotoxinaktivität von 3OO EU/dl bzw. 1OOO EU/dl erreicht wurde. Nach Endotoxinzugabe wurde der Ansatz dann 6O Minuten bei 37°C inkubiert. Nach der Inkubation wurde die Endotoxinaktivität in der Probe mit dem Limulus-Test quantitativ erfaßt.

Es zeigte sich, daß die Endotoxinaktivität im Plasma durch den Zusatz von Apotransferrin-Zink oder Apotransferrin-Kupfer im Vergleich zu den Proben, denen das entsprechende Albumin zugesetzt wurde, signifikant herabgesetzt wurde.
a) Bei Inkubation von Endotoxin mit Albumin-Zink fand sich sowohl bei einem Zinkgehalt von 4,8µg pro Gramm Albumin (Zn(A)) wie auch bei einem Zinkgehalt von 598 µg pro Gramm Albumin (Zn(B)) nach der Inkubation eine Endotoxinaktivität im Plasma, die maximal ca. 4% niedriger war, als die Aktivität der zugegebenen Endotoxinmenge. Eine annähernd ähnliche Reduzierung der Endotoxinaktivität um ca. 4% fand sich nach Inkubation mit Albumin-Kupfer, das einen Kupfergehalt von 74O µg Kupfer pro Gramm Albumin hatte.
b) durch Zusatz von Atf.-Zn(A) wurde die Endotoxinaktivität im Plasma bei einer Ausgangsendotoxinkonzentration von 3OO EU/dl Endotoxin um 83,4 ± 4,2% ( n = 20 ) und bei 1OOO EU/dl um 69,1% ± 6,8% ( n= 18 ) gesenkt.
c) Bei Zusatz von Apotransferrin-Zink (Atf.-Zn(B)) mit einem Zinkgehalt von 598 µg Zink pro Gramm Apotransferrin zu dem Plasma betrug die Abnahme der Endotoxinaktivität nach Inkubation mit 5OO EU/dl Endotoxin: 93,9% ± 2,9% (n = 20) und nach Inkubation mit 1OOO EU/dl Endotoxin: 94,8 % ± 2,7% (n = 2O).
d) Bei Zusatz von Apotransferrin-Kupfer (Atf.-Cu(B)) mit einem Kupfergehalt von 74O µg Kupfer pro Gramm Apotransferrin zu dem Plasma betrug die Abnahme der Endotoxinaktivität nach Inkubation mit 5OO EU/dl Endotoxin: 92,7% ± 4,1% ( n = 16 ) und nach Inkubation mit 1OOO EU/dl Endotoxin: 90,8 % ± 3,4% ( n = 16 ).

### Zusammenfassende Beurteilung der Ergebnisse:

1.) Die Komplexe von Apotransferrin mit zweiwertigen Zink- oder Kupfer-Ionen sind in der Lage, konzentrationsabhängig die biologische Aktivität des Endotoxins herabzusetzen und eignen sich damit auch sehr gut für die Bekämpfung der toxischen Wirkung des Endotoxins im Organismus.
2.) Apotransferrin, welches frei von Metallkationen ist, hat nur eine schwache Wirkung auf die Endotoxinaktivität.
3.) Das Ausmaß, in dem durch Apotransferrin-Zink oder Apotransferrin-Kupfer die toxische Wirkung des Endotoxins herabgesetzt wird, nimmt mit dem Gehalt des Apotransferrins an Zink oder Kupfer zu. Der Gehalt des Apotransferrins an Zink oder Kupfer sollte hierbei mindestens O,1 µg pro Gramm Apotransferrin betragen.
4.) Die Verwendung von Apotransferrin, das zweiwertige Zink- oder Kupfer-Ionen anstelle der dreiwertigen Eisen-Ionen angelagert hat, zur Bekämpfung der toxischen Wirkung des Endotoxins bietet bei annähernd ähnlicher Neutralisationskapazität für Endotoxin den Vorteil, daß bei der Applikation dieser Apotransferrin-Metall-Komplexe nicht die Bildung von toxischen Sauerstoffradikalen im Organismus begünstigt wird.
5.) Sowohl die Zink-Ionen, die mit dem Apotransferrin-Zink-Komplex zugeführt werden, wie auch die Kupfer-Ionen des Apotransferrin-Kupfer sind in der Lage, das Enzym " Superoxid-Dismutase" sowie eine Reihe von Peroxidasen zu aktivieren. Dadurch bietet die Applikation von Apotransferrin-Zink oder Apotransferrin-Kupfer im Vergleich zu anderen therapeutischen Maßnahmen den entscheidenden Vorteil, daß zusätzlich zu der Neutralisation von Endotoxin auch noch eine verbesserte Elimination der toxischen Sauerstoffverbindungen erreicht wird, die bei der Endotoxinämie verstärkt gebildet werden.
6.) Die Verwendung von Apotransferrin-Zink zur Endotoxin-Neutralisation bietet außerdem den Vorteil, daß durch diese therapeutische Maßnahme nicht nur die Neutralisation von Endotoxin erreicht wird, sondern daß zusätzlich auch die Proteinsynthese und damit u.a. eventuell ablaufende Wundheilungsprozesse begünstigt werden, da Zink-Ionen die DNA- und RNA-Synthese fördern.
7.) Die Apotransferrin-Zink oder Apotransferrin-Kupfer Komplexe können mit Plasma-Proteinlösungen oder Immunglobulin-Lösungen kombiniert werden, wobei die Immunglobulin-Lösungen entweder nur IgG oder aber auch die anderen Plasma-Proteine, insbesondere IgM und IgA enthalten können.
8.) Durch die Kombination von Apotransferrin-Zink und Apotransferrin-Kupfer mit anderen Plasmaproteinen, insbesondere mit Immunglobulinen der IgG- und IgM-Fraktion wird eine signifikant höhere Reduzierung der biologischen Aktivität des Endotoxins erreicht, als es die einzelnen Komponenten allein bewirken können.
9.) Durch den Synergismus bei der Neutralisation der Endotoxine ist die Kombination von Apotransferrin-Zink und Apotransferrin-Kupfer für therapeutische Maßnahmen zur Bekämpfung der toxischen Wirkung von Endotoxin bei allen krankhaften Veränderungen besonders geeignet, bei denen es zu einem stärkeren und länger andauernden Endotoxinübertritt in die Blutbahn kommt.

### Die Erfindung wird durch folgende Beispiele erläutert.

### BEISPIEL : 1

Zur Objektivierung der Bedeutung, die der Gehalt des Apotransferrins an zweiwertigen Zink- oder Kupfer-Ionen für dessen Fähigkeit zur Inaktivierung von Endotoxin im Plasma hat, wurden Untersuchungen mit dem Apotransferrin-Zink durchgeführt:

Humanplasma von gesunden Spendern wurde mit O,9% NaCl 1:1O verdünnt und danach durch Erhitzen ( 8O°C, 5 Min. ) inaktiviert. Anschließend wurde Apotransferrin-Zink dem Plasma zugesetzt, welches einen unterschiedlichen Zinkgehalt hatte. Die Apotransferrinkonzentration betrug im Ansatz jeweils 3OO mg/dl. Durch Zugabe von unterschiedlichen Mengen ZnCl₂ war der Zinkgehalt des Apotransferrins so eingestellt worden, daß in der Lösung ein Mol-Verhältnis zwischen Apotransferrin und Zink von 28 : 1, 1 : 1, und 1 : 3 bestand.
Nach der Zugabe von Apotransferrin-Zink zu dem inaktivierten Plasma wurde Endotoxin von E. coli den Plasmaproben in unterschiedlichen Konzentrationen zugesetzt, sodaß im Ansatz Endotoxinkonzentrationen von 5O, 1OO, 2OO, 3OO, 5OO, 75O, 1OOO und 15OO EU/dl erreicht wurden. Sofort nach der Endotoxinzugabe wurde der Ansatz dann 6O Minuten bei 37°C inkubiert. Danach wurde die Endotoxinrestaktivität bestimmt, die in den Proben noch nachweisbar war. Aus diesen Werten wurde die Inaktivierungskapazität der verschiedenen Apotransferrin-Zink-Lösungen für einen Endotoxinüberschuß von 1O EU/dl berechnet.

Für einen Endotoxinüberschuß im Reaktionsansatz von 1O EU/dl wurden in Abhängigkeit von dem jeweiligen Molverhältnis zwischen Apotransferrin und Zink folgende Werte für die Inaktivierungskapazität von jeweils 1OO mg/dl Apotransferrin-Zink gefunden:
a) 28 : 1 : 19,2 EU pro 1OO mg Apotransferrin-Zink
b) 1 : 1 : 87,1 EU pro 1OO mg Apotransferrin-Zink
c) 1 : 3 : 279,8 EU pro 1OO mg Apotransferrin-Zink

Durch die Erhöhung des Zinkgehaltes der Apotransferrinlösung gelingt es somit, die Inaktivierungskapazität zu steigern. Hierbei führt beispielsweise die Erhöhung des Zinkgehaltes von einem Molverhältnis zwischen Apotransferrin und Zink, das 28 : 1 beträgt, auf ein Molverhältnis von 1 : 3 zu einer Steigerung der Inaktivierungskapazität um einen Faktor von 14,5. Bei einem Molverhältnis von 1 : 3 ist zu erwarten, daß die meisten Apotransferrinmoleküle zwei Zink-Ionen angelagert haben.

### BEISPIEL 2

Entsprechend den Bedingungen, wie sie bei der Therapie von gramnegativen Infektionen mit Antibiotika zu finden sind, wurde im Tierversuch ein Endotoxinanstieg im Blut dadurch induziert, daß zunächst Bakterien in die Bauchhöhle gegeben werden und durch die anschließende intravenöse Gabe eines bakteriziden Antibiotikums ein rascher Zerfall dieser Bakterien ausgelöst wird.
Hierzu wurde narkotisierten Wistar-Ratten (25O - 3OO g) zur Blutabnahme zunächst ein Katheter in die rechte Halsvene (Vena Jugularis ) eingelegt. Anschließend wurde eine definierte Zahl verschiedener Bakterien ( E.coli, Klebsiella species und Pseudomonas aeruginosa; in einer Konzentration von jeweils 7.5 x 1O⁵ cfu/kg, d.h. insgesamt 2.3 x 10⁶ cfu/kg ) in die Bauchhöhle appliziert. 3O Minuten nach Gabe der Bakterien in die Bauchhöhle wurde über den Venenkatheter mit einem Perfusor langsam das zu prüfende Präparat (Apotransferrin-Zink (Atf.-Zn(B)) sowie Apotransferrin-Zink in Kombination mit Immunglobulin ) in einer Dosierung von 25O mg/kg Körpergewicht intravenös verabreicht. Die Kontrolltiere erhielten Albumin-Zink in entsprechender Dosierung und entsprechender Zink-Konzentration. Zur Erzeugung einer Endotoxinämie wurde eine Stunde nach Bakteriengabe ein bakterizides Antibiotikum (IMIPENEM = Zienam^{R}) intravenös appliziert. Die Blutabnahmen zur Bestimmung der Endotoxinaktivität und der Bakterienzahl erfolgten vor, sowie - in 6O minütigem Abstand - nach der Bakteriengabe über insgesamt 6 Stunden.

Folgende Präparationen wurden i.v. verabreicht:
Apotransferrin((Atf.-Zn(B)) mit einem Zinkgehalt von 598 µg Zn²⁺ pro Gramm; sowie die Kombination von Apotransferrin-Zink (Atf.-Zn(B)) mit dem IgM(12%)-angereichertem 7S-IgG.

### Ergebnisse:

a) Bei i.v.-Gabe von Albumin-Zink steigt die Endotoxinaktivität im Plasma nach Applikation des Antibiotikums sehr stark an. Bereits eine Stunde nach Gabe des Antibiotikums wird eine mittlere Endotoxinaktivität von 234 EU/dl erreicht. Fünf Stunden nach Gabe des Antibiotikums beträgt die Endotoxinaktivität im Plasma 278 ± 31 EU/dl.
b) Durch i.v.-Gabe von Apotransferrin-Zink( Atf.-Zn(B) ) wird die initiale Zunahme der Endotoxinaktivität im Plasma - eine Stunde nach Gabe des Antibiotikums - im Vergleich zur Albumin-Kontrollgruppe um ca. 68,3 % ± 7,2% reduziert. Bei Versuchsende, d.h. fünf Stunden nach Gabe des Antibiotikums ist die Endotoxinaktivität bei der Gruppe, die Atf.-Zn(B) erhalten hat, noch um ca. 63,5 % ± 6,5% niedriger, als bei der Albumin-Gruppe.
c) Bei i.v.-Gabe von Apotransferrin-Zink( Atf.-Zn(B) ) in Kombination mit einem Immunglobulin( IgG/A/M ), welches mit 12% IgM angereichert wurde, ist die initiale Zunahme der Endotoxinaktivität im Plasma - eine Stunde nach Gabe des Antibiotikums - um ca. 73 % niedriger, als bei der Albumin-Kontrollgruppe. Fünf Stunden nach Gabe des Antibiotikums ist die Endotoxinaktivität bei der Gruppe, welche diese Kombination erhalten hat, ca. 83,2% ± 7,9 % niedriger, als bei der Albumin-Gruppe.

Die Steigerung der Inaktivierungskapazität durch die Kombination von Apotransferrin-Zink mit Immunglobulin bietet die Möglichkeit, auch bei einem längerer andauernden Endotoxineinstrom einen stärkeren Anstieg der Endotoxinaktivität im Plasma zu beherrschen. Die alleinige Gabe von Apotransferrin-Zink( Atf.-Zn(B) ) führt in der Initialphase zu einer ähnlich starken Reduzierung der Endotoxinaktivität, wie die Kombination von Apotransferrin-Zink mit Immunglobulin. Doch ohne Erhöhung der Dosis oder des Zinkanteils reicht die Inaktivierungskapazität bei alleiniger Gabe von Apotransferrin-Zink bei einem kontinuierlichen starken Endotoxinübertritt in das Blut nach einer gewissen Zeit nicht mehr aus, wodurch es wieder zu einem Ansteigen der Endotoxinaktivität im Plasma kommen kann. Die Kombination von Immunglobulin mit Apotransferrin-Zink stellt hier, auch bei Verwendung von Apotransferrin-Zink mit einem Zinkgehalt unter 5OO µg Zink pro Gramm Apotransferrin eine therapeutisch sehr wirkungsvolle Alternative dar.

## Patentansprüche

1. Verwendung eines Mittels, bestehend aus Apotransferrin, an welches zweiwertige Zink- oder Kupfer-Kationen angelagert sind, zur Herstellung eines Arzneimittels zur Bekämpfung der toxischen Wirkung des Endotoxins bei Endotoxinämie.

2. Verwendung eines Mittels nach Anspruch 1, dadurch gekennzeichnet, daß das Apotransferrin-Molekül ein oder zwei Zink- oder Kupfer-Kationen gebunden besitzt.

3. Verwendung eines Mittels nach einem der vorangehenden Ansprüche, dadurch gekennzeichnet, daß der Gehalt des Apotransferrin-Metall-Komplex an einem dieser Übergangsmetalle mindestens bis 0,1 µg pro Gramm Apotransferrin beträgt.

4. Verwendung eines Mittels nach einem der vorangehenden Ansprüche, dadurch gekennzeichnet, daß die Apotransferrin-Zink-Komplexe oder die Apotransferrin-Kupfer-Komplexe mit Proteinen kombiniert werden.

5. Verwendung eines Mittels nach Anspruch 4, dadurch gekennzeichnet, daß diese Proteine Plasma-Proteine sind.

6. Verwendung eines Mittels nach Anspruch 5, dadurch gekennzeichnet, daß dieses Plasma-Protein ein Transferrin ist.

7. Verwendung eines Mittels nach Anspruch 5, dadurch gekennzeichnet, daß diese Plasma-Proteine Immunglobuline sind.

8. Verwendung eines Mittels nach einem der vorangehenden Ansprüche, dadurch gekennzeichnet, daß als Immunglobuline IgG und IgM oder IgA eingesetzt werden.

9. Verwendung eines Mittels nach Anspruch 7 oder 8, dadurch gekennzeichnet, daß das Apotransferin-Zink oder Apotransferin-Kupfer zu Immunglobuline-Lösungen zugesetzt wird.

10. Verwendung eines Mittels nach einem der vorangehenden Ansprüche, dadurch gekennzeichnet, daß Apotransferin-Kupfer in einer Plasma-Proteinlösung angereichert sind.

11. Verwendung eines Mittels nach einem der vorangehenden Ansprüche, dadurch gekennzeichnet, daß die Plasma-Proteinlösung eine Serum-Konserve ist.

## Claims

1. Use of a substance consisting of apotransferrin that has bound divalent zinc or copper cations for the manufacture of a substance to combat the toxic effects of endotoxins in case of endotoxemia.

2. Use of a substance according to claim 1 characterized in that the apotransferrin molecule has bound one or two zinc or copper cations.

3. Use of a substance according to one of the preceding claims characterized in that the content of one of the above transition metals in the apotransferrin-metal complex is at least 0.1 µg per gram apotransferrin.

4. Use of a substance to one of the preceding claims characterized by the combination of apotransferrin-zinc complexes or apotransferrin-copper complexes with proteins.

5. Use of a substance according to claim 4 characterized by use of plasma proteins as the proteins.

6. Use of a substance according to claim 5 characterized by use of transferrin as the plasma protein.

7. Use of a substance according to claim 5 characterized by use of immunoglobulins as the plasma proteins.

8. Use of a substance according to claim 7 characterized by use of IgG and IgM or IgA as immunoglobulins.

9. Use of a substance according to claims 7 or 8 characterized by addition of the apotransferrin-zinc or apotransferrin-copper to immunoglobulin solutions.

10. Use of a substance according to one of the preceding claims, characterized by enrichment of apotransferrin-zinc or apotransferrin-copper in a plasma protein solution.

11. Use of a substance according to claim 10 characterized by the plasma protein solution being in the form of serum preserve.

## Revendications

1. Utilisation d'un agent composé d'apotransferrine sur laquelle sont fixés des cations bivalents de zinc ou de cuivre pour la production d'un médicament destiné à combattre l'effet toxique de l'endotoxine lors d'endotoxinémie.

2. Utilisation d'un agent, selon la revendication 1, caractérisée par le fait que la molécule d'apotransferrine possède un ou deux cations de zinc ou de cuivre liés.

3. Utilisation d'un agent, selon l'une des revendications précédentes, caractérisée par le fait que la teneur du complexe apotransferrine-métal de l'un de ces métaux intermédiaires s'élève à au moins 0,1 µg par gramme d'apotransferrine.

4. Utilisation d'un agent, selon l'une des revendications précédentes, caractérisée par le fait que les complexes apotransferrine-zinc ou apotransferrine-cuivre sont combinés avec des protéines.

5. Utilisation d'un agent, selon la revendication 4, caractérisée par le fait que ces protéines sont des protéines de plasma.

6. Utilisation d'un agent, selon la revendication 5, caractérisée par le fait que la protéine de plasma citée ci-dessus est une transferrine.

7. Utilisation d'un agent, selon la revendication 5, caractérisée par le fait que ces protéines de plasma sont des immunoglobulines.

8. Utilisation d'un agent, selon l'une des revendications précédentes, caractérisée par le fait que l'on utilise des immunoglobulines IgG et IgM ou IgA.

9. Utilisation d'un agent, selon la revendication 7 ou 8, caractérisée par le fait que l'on ajoute l'apostransferrine-zinc ou l'apostransferrine-cuivre à des solutions d'immunoglobulines.

10. Utilisation d'un agent, selon l'une des revendications précédentes, caractérisée par le fait que l'apostransferrine-cuivre est enrichie dans une solution de protéines de plasma.

11. Utilisation d'un agent, selon l'une des revendications précédentes, caractérisée par le fait que la solution de protéines de plasma est un sachet de sérum.
